# EUROPÄISCHE PATENTSCHRIFT

(11) **EP 0 670 331 B1**
(45) Veröffentlichungstag und Bekanntmachung des Hinweises auf die Patenterteilung: **05.11.2003**
(21) Anmeldenummer: 95102198.9
(22) Anmeldetag: 17.02.1995
(51) Int. Cl.: C07K 14/745, C07K 1/113, G01N 33/68

(54) **Verfahren zur Reaktivierung von gereinigten Membranproteinen durch Einfrieren**
Process to reactivate purified membrane proteins by freezing
Procédé de réactivation de protéines de membrane purifiées par congélation

(30) Priorität: 05.03.1994 DE 4407386
(43) Veröffentlichungstag der Anmeldung: 06.09.1995
(73) Patentinhaber: Dade Behring Marburg GmbH, 35001 Marburg (DE)
(72) Erfinder: Zander, Norbert F., Dr., D-35039 Marburg (DE)

(56) Entgegenhaltungen:
- BIOLOGICAL ABSTRACTS, vol. 76, no. 10 1983, Philadelphia, PA, US; abstract no. 71791, J R BROTHERUS ET AL. 'Soluble and enzymatically stable (Na+, K+) ATPase from mammalian kidney consisting predominantly of protomer alpha-beta units; preparation, assay and reconstitution of active sodium, potassium transport' Seite 7890 ; & BIOCHIM. BIOPHYS. ACTA, Bd.731, Nr.2, 1983 Seiten 290 - 303
- CHEMICAL ABSTRACTS, vol. 104, no. 3, 20. Januar 1986, Columbus, Ohio, US; abstract no. 18027q, D DE PROST ET AL. 'Quantitative assessment of procoagulant activity in isolated rat glomeruli' Seite 342 ; & KIDNEY INT., Bd.28, Nr.3, 1985 Seiten 566 - 568
- JOURNAL OF BIOLOGICAL CHEMISTRY., Bd.267, Nr.3, 25. Januar 1992, BALTIMORE US Seiten 1674 - 1679 T SASAKI ET AL. 'Molecular mechanism of regulation of Ca2+ pump ATPase by phospholamban in cardiac sarcoplasmic reticulum'
- CHEMICAL ABSTRACTS, vol. 121, no. 19, 7. November 1994, Columbus, Ohio, US; abstract no. 225786c, P B CONTINO ET AL. 'Use of an oriented transmembrane protein to probe the assembly of a supported phospholipid bilayer' Seite 566 ; & BIOPHYS. J., Bd.67, Nr.3, Juni 1994 Seiten 1113 - 1116
- BACH ET AL BIOCHEMISTRY Bd. 25, 1986, Seiten 4007 - 4020

## Beschreibung

Die Erfindung betrifft ein Verfahren zur Reaktivierung von gereinigten Membranproteinen zu aktiven Proteinen.

Membranproteine, z. B. Rezeptoren, bestehen aus.einer oder mehreren Transmembrandomänen sowie intra- und extrazellulären Domänen. Die Aktivität solcher Proteine wird häufig nach Integration des aufgereinigten Proteins in eine künstliche Membran gemessen.

Als Beispiel kann der Gewebsfaktor (Gewebs-Thromboplastin) gelten. Dieser Rezeptor für den Faktor VII besteht aus Apoprotein sowie Lipiden. Das Apoprotein ist ein glykosyliertes Polypeptid von 263 Aminosäuren. Nahe dem carboxy-terminalen Ende besitzt es eine hydrophobe Sequenz von 23 Aminosäuren, mit der es in der Membran verankert ist. Der intrazelluläre Anteil besteht aus 21 Aminosäuren. In vivo kommt der Gewebsfaktor als integrales Membranprotein von Zellen vor, die nicht in direktem Kontakt mit Blut stehen. Seine physiologische Funktion besteht darin, als Zelloberflächenrezeptor bei Kontakt mit Blut bzw. Plasma den plasmatischen Gerinnungsfaktor VII zu binden und zu aktivieren. Dieser Komplex besitzt Serinprotease-Aktivität und ist seinerseits in der Lage, die Faktoren IX und X zu aktivieren und die Gerinnung auszulösen.

Bei der Isolation von Gewebsfaktor können zwei Methoden unterschieden werden. Bei der einen wird der aktive Gewebsfaktor teilweise gereinigt, indem geeignete Gewebe aufgeschlossen und die entsprechende Membranfraktion isoliert wird. Verwendung findet dieses Material in erster Linie bei der Herstellung von diagnostisch eingesetzten Reagenzien zur Überprüfung der plasmatischen Blutgerinnung. Da das Membranprotein als ganzes (d.h. inklusive gebundener Lipidmoleküle) isoliert wird, ist eine Reaktivierung des Apoproteins nicht nötig.

Bei dem anderen Verfahren wird das isolierte Apoprotein gewonnen. Da es fast keine Aktivität mehr besitzt, muß es reaktiviert werden. Hierzu ist es erneut in eine Lipidmembran zu integrieren. Unumgänglich ist dieses Verfahren für Protein, das auf rekombinantem Weg gewonnen wird, da die mikrobiologischen Organismen, die zur Produktion eingesetzt werden, in der Regel nicht in der Lage sind, ein ausreichend aktives Membranprotein zu liefern. Grundsätztlich gelten diese Überlegungen auch für Proteine, die durch Teil- oder Totalsynthese in vitro hergestellt werden.

Zum Wiedereinbau aufgereinigter Membranproteine in eine Lipidmembran sind eine Reihe von Verfahren bekannt. Üblicherweise werden dazu Phospholipide mit einem Detergenz, z. B. Triton-X 100, in eine wäßrige Lösung gebracht. Diese wird anschließend mit dem Membranprotein gemischt. Sodann wird das Detergenz entfernt, z.B. durch Dialyse. Beim Mischen des Apoproteins mit der Phospholipidlösung bzw. bei der Entfernung des Detergenzes erfolgt der Einbau des Proteins in die sich bildenden Membranvesikel. Desoxycholat ist das bevorzugte Detergenz, da es durch Dialyse entfernt werden kann. Prinzipiell sind andere Detergenzien jedoch auch einsetzbar, wenn man sie wieder aus dem Ansatz entfernen kann.

Brotherus et al. (Biochimica et Biophysica Acta, 731 (1983) 290-303) beschreiben den Einbau einer mittels Detergenz aus der Zellmembran isolierten (Na⁺+K⁺)-ATPase in Phospholipidvesikel, wobei das Enzym zusammen mit Phosphatidylcholin in flüssigen Stickstoff schockgefroren, nach dem Auftauen bei Raumtemperatur mit Ultraschall behandelt und das die Phospholipidvesikel umgebende Medium ausgetauscht wird.

Der vorliegenden Erfindung lag die Aufgabe zugrunde, ein vereinfachtes Verfahren zur Verfügung zu stellen, mit dessen Hilfe Membranproteine mit einer minimalen Menge von Schritten und möglichst schnell in Lipidvesikel integriert und somit reaktiviert werden können.

Überraschenderweise wurde gefunden, daß durch einfaches Einfrieren eines Gemischs von Phospholipiden, Detergenz und Membranprotein diese Relipidisierung erreicht werden kann. Eine nachträgliche Entfernung des Detergenzes aus dem Reaktionsgemisch ist nicht unbedingt nötig.

Grundsätzlich kann das erfindungsgemäße Verfahren auch auf Mischungen gereinigter Proteine angewendet werden.

Es wurde gefunden, daß die Relipidisierung erreicht werden kann, indem ein vorzugsweise konzentriertes flüssiges, vorzugsweise wässriges Gemisch von Phospholipid, Detergenz und Membranprotein eingefroren wird. Das Einfrieren kann bei Temperaturen von 0°C bis -200°C, bevorzugterweise bei etwa -70°C, erfolgen. Die Geschwindigkeit des Einfrierens ist unkritisch. Die Zeit, während der das Gemisch im gefrorenen Zustand verbleibt, kann zwischen 1 Stunde und 1 Jahr variieren; bevorzugterweise wird das Gemisch für etwa 16 Stunden eingefroren. Es ist zweckmäßig, das gesamte Gemisch homogen durchzufrieren. Das Auftauen erfolgt bei 0°C bis 100°C, bevorzugterweise bei etwa 37°C.

Als Phospholipid kann ein dem Fachmann an sich bekanntes Material pflanzlichen oder tierischen Ursprungs als natürliche Mischung bekannter Komponenten verwendet werden. Ebenso einsetzbar sind dem Fachmann bekannte reine Phospholipide oder Mischungen davon.

Bevorzugterweise wird in dem erfindungsgemäßen Verfahren mit pflanzlichen Phospholipidgemischen gearbeitet. Die Lipidkonzentration beträgt im einzufrierenden Gemisch vorzugsweise zwischen >0 bis 500 mg/ml.

Das Membranprotein kann humanen, pflanzlichen, tierischen, mikrobiellen oder rekombinanten Ursprungs sein. Ebenfalls möglich ist die Aktivierung von inaktiven Mutanten von natürlich vorkommenden Proteinen.

Bevorzugt ist als ein solches Protein ein humanes und gegebenenfalls rekombinantes Protein.

Bevorzugt ist die Verwendung eines gelösten Membranproteins in einer Konzentration von >0 bis 50 mg/ml.

Als Detergenz können nichtionische, anionische oder kationische Detergenzien in reiner Form oder in Mischung eingesetzt werden. Die Konzentration liegt zwischen >0% und 50 %. Beim erfindungsgemäßen Verfahren wird vorzugsweise mit dem nichtionischen Detergenz Triton-X 100 in einer Konzentration von 2 % gearbeitet.

Vorzugsweise wird zunächst Phospholipid in Wasser oder einer wäßrigen Flüssigkeit in einer Konzentration von >0% bis 50 % emulgiert. Detergenz wird in Wasser oder einer wäßrigen Flüssigkeit in einer Konzentration von >0% bis 50 % gelöst. Nun werden Phospholipid, Detergenz und Membranprotein im jeweilig geeigneten Mengenverhältnis gemischt. Das Gemisch wird bei 0°C bis -200°C eingefroren. Nachdem der gesamte Ansatz durchgefroren ist, verbleibt er eine Zeit zwischen 1 Stunde und 1 Jahr, vorzugsweise 16 Stunden, bei dieser Temperatur. Anschließend wird er bei einer Temperatur zwischen 0°C und 100°C, vorzugsweise bei 37°C, aufgetaut. Das überschüssige Detergenz kann durch Dialyse gegen Puffer entfernt oder durch Zugabe einer großen Menge Puffer so verdünnt werden, daß es die folgenden Reaktionen nicht mehr stört.

Vorzugsweise wird in Gegenwart von Wasser eingefroren. Es erscheint jedoch auch möglich, ein flüssiges Gemisch der Komponenten ohne Anwesenheit von Wasser einzufrieren. Auch könnte das Membranprotein beim Einfrieren mit Lipid und Detergenz an eine feste Matrix gebunden sein

Nach der Relipidisierung liegt das Membranprotein in aktiver Form eingebaut in eine Lipidmembran vor. Es kann zur weiteren Verwendung gegebenenfalls mit Zusätzen versehen und konfektioniert werden.

Wird beispielsweise Gewebsfaktor-Apoprotein im erfindungsgemäßen Verfahren relipidisiert, ist eine Verwendung als Therapeutikum oder als Diagnostikum möglich. Im zweiten Fall kann der relipidisierte Gewebsfaktor insbesondere zu einem Reagenz zur Bestimmung der Prothrombinzeit zum Zwecke der Überprüfung der plasmatischen Blutgerinnung verarbeitet werden.

Gegenüber den bekannten und beschriebenen Verfahren zur Relipidisierung von Membranproteinen liegt der Vorteil des erfindungsgemäßen Verfahrens in seiner einfacheren Durchführbarkeit. Insbesondere kann der arbeitsaufwendige und kostspielige Arbeitsschritt der Dialyse gegen detergenzfreien Puffer entfallen.

Am Beispiel des Gewebsfaktor-Apoproteins soll die Erfindung näher erläutert werden.

### Beispiele

### Beispiel 1:

### Relipidisierung eines rekombinant gewonnenen und gereinigten humanen Gewebsthromboplastin-Apoproteins durch Einfrieren

Es wurden gemischt:

| | | |
|---|---|---|
| 30 ml | 10 % | Phospholipidsuspension in aqua dest. (Phospholipon 25P,Fa. Nattermann) |
| 5 ml | 1 mg/ml | gereinigtes humanes Gewebsthromboplastin-Apoprotein |
| 4 ml | 20 % | Triton-X100 |
| 1 ml | | aqua dest. |

Nach einstündigem Rühren wird der Ansatz 16 Stunden lang bei -70° eingefroren.
Nach dieser Zeit wird der Ansatz bei 37°C aufgetaut und mit 201:

| | |
|---|---|
| 50 mM | N-(2-Hydroxyethyl)piperazin-N'-(2-Ethansulfonsäure) pH 7.5 |
| 13 mM | Calciumchlorid |

verdünnt.
Die Bestimmung der Prothrombinzeit erfolgte an einem Koagulometer nach Schnitger und Groß (0.1 ml humaner Normalplasmapool + 0.2 ml relipidisierter Gewebsfaktor) und ergab eine Gerinnungszeit von 10.8 s .

### Beispiel 2:

### Temperaturabhängigkeit der Relipidisierung

Reagenzien wurden grundsätzlich wie in Beispiel 1 beschrieben hergestellt. Das Gemisch von Membranprotein, Detergenz und Lipid wurde bei -20°C und -60°C eingefroren und anschließend mit Puffer verdünnt.
Als Negativkontrollen wurden Gemische von Lipid und Protein bzw. Lipid und Detergenz in gleicher Weise eingefroren und aufgetaut. Anschließend wurde die noch fehlende Komponente und Puffer zugegeben und gemischt.
An einem'Koagulometer nach Schnitger und Groß ergaben sich mit einem humanen Normalplasmapool die folgenden Gerinnungszeiten:

**Tabelle I**

| **Temperaturabhängigkeit des Einfrierschritts** | | | |
|---|---|---|---|
| **erfindungsgemäß** | **Temperatur** | **Zusätze** | **Gerinnungszeit** |
| | | | |
| Lipid/Detergenz/Protein | -20°C | Puffer | 9.5 s |
| | | | |
| Lipid/Detergenz/Protein | -60°C | Puffer | 9.5 s |
| | | | |

| **Negativkontrollen** | | | |
|---|---|---|---|
| | | | |
| Lipid/Protein | -60°C | Detergenz/Puffer | 21.0 s |
| | | | |
| Lipid/Detergenz | -60°C | Protein/Puffer | 18.7 s |

### Beispiel 3:

### Qualität der Reagenzien: Faktor VII-Empfindlichkeit

Ein humaner Normalplasmapool wurde mit einem Faktor VII-Mangelplasma (Behringwerke AG, Marburg, Germany) verdünnt. Es wurden Faktor VII- Konzentrationen zwischen 100 % und 1 % (bezogen auf den humanen Normalplasmapool) eingesetzt.

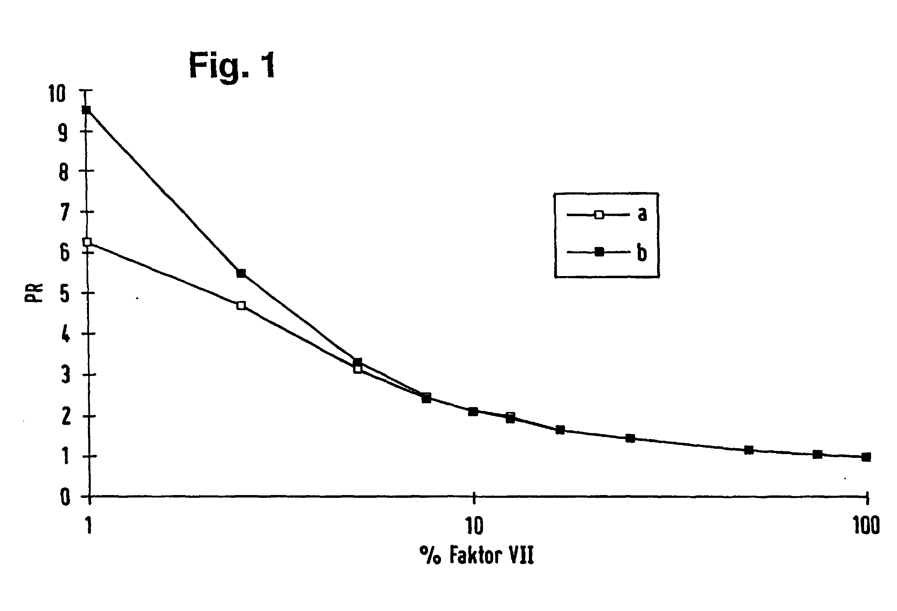

Die Prothrombinzeiten der Proben wurden anschließend an einem Koagulometer nach Schnitger und Groß bestimmt. Die gemessenen Zeiten wurden ins Verhältnis zu der Prothrombinzeit des humanen Normalplasmapools gesetzt. Fig. 1 zeigt die relativen Prothrombinzeiten [Prothrombinratio PR = (PT)/(PT _{100% Faktor VII})] in Abhängigkeit von der Faktor VII-Konzentration der Probe. Es wurden zwei Gewebsfaktor-Reagenzien verwendet:
- a: nativer Gewebsfaktor, isoliert aus menschlicher Plazenta (^{R}Thromborel S, Behringwerke AG)
- b: Reagenz ausgehend von rekombinanten Gewebsfaktor-Apoprotein, hergestellt nach dem erfindungsgemäßen Verfahren (gemäß Beispiel 1).

Der Normalbereich der Faktor VII- Konzentration in einer menschlichen Population (95-Perzentil) wird durch Prothrombin-Ratios von 1.00 ± 0.20 erfaßt. Plasmen, die eine außerhalb dieses Bereichs liegende Ratio aufweisen, werden als pathologisch erkannt. Die dieser Empfindlichkeitsgrenze entsprechende Faktor VII-Konzentration ist für die beiden hier untersuchten.
Reagenzien sehr ähnlich.
Im Bereich unter 10 % Faktor VII ist das Reagenz auf der Basis des rekombinanten Gewebsfaktor-Apoproteins deutlich empfindlicher.

## Patentansprüche

1. Verfahren zur Reaktivierung von Gewebsfaktor-Apoprotein, **dadurch gekennzeichnet, daß** Gewebsfaktor-Apoprotein in Gegenwart eines Lipids und eines Detergenzes eingefroren und das Gefrorene wieder aufgetaut wird und das Reaktionsgemisch, ohne dass das Detergenz nachträglich entfernt wird, mit Puffer verdünnt wird und gegebenenfalls mit Zusätzen versehen zur weiteren Verwendung als Therapeutikum oder als Diagnostikum konfektioniert wird.

2. Verfahren nach Anspruch 1, **dadurch gekennzeichnet, daß** ein reines Lipid, eine Mischung reiner Lipide oder eine natürliche Lipidmischung verwendet wird.

3. Verfahren nach Anspruch 1, **dadurch gekennzeichnet, daß** das Detergenz in einer Konzentration bis 50 % verwendet wird.

4. Verfahren nach Anspruch 1, **dadurch gekennzeichnet, daß** ein nichtionisches Detergenz verwendet wird.

5. Verfahren nach Anspruch 1, **dadurch gekennzeichnet, daß** bei einer Temperatur von 0°C bis -200°C eingefroren wird.

6. Verfahren nach Anspruch 1, **dadurch gekennzeichnet, daß** gelöstes Gewebsfaktor-Apoprotein verwendet wird.

7. Verfahren nach Anspruch 1, **dadurch gekennzeichnet, daß** die Konzentration des Gewebsfaktor-Apoproteins bis 50 mg/ml beträgt.

8. Verfahren nach Anspruch 1, **dadurch gekennzeichnet, daß** die Lipidkonzentration bis 500 mg/ml beträgt.

9. Verfahren nach Anspruch 1, **dadurch gekennzeichnet, daß** das Gewebsfaktor-Apoprotein menschlichen, tierischen oder rekombinanten Ursprungs ist.

10. Verfahren nach Anspruch 1, **dadurch gekennzeichnet, daß** das Gewebsfaktor-Apoprotein eine Mutante des Gewebsfaktor-Apoproteins ist.

## Claims

1. A process for reactivating tissue factor apoprotein, wherein tissue factor apoprotein is frozen in the presence of a lipid and a detergent and the frozen material is thawed once again and, without the detergent subsequently being removed, the reaction mixture is diluted with buffer and, where appropriate provided with additives, formulated for further use as a therapeutic agent or as a diagnostic agent.

2. The process as claimed in claim 1, wherein a pure lipid, a mixture of pure lipids or a natural lipid mixture is used.

3. The process as claimed in claim 1, wherein the detergent is used at a concentration of up to 50%.

4. The process as claimed in claim 1, wherein a nonionic detergent is used.

5. The process as claimed in claim 1, wherein freezing takes place at a temperature of from 0°C to -200°C

6. The process as claimed in claim 1, wherein dissolved tissue factor apoprotein is used.

7. The process as claimed in claim 1, wherein the concentration of the tissue factor apoprotein is up to 50 mg/ml.

8. The process as claimed in claim 1, wherein the. lipid concentration is up to 500 mg/ml.

9. The process as claimed in claim 1, wherein the tissue factor apoprotein is of human, animal or recombinant origin.

10. The process as claimed in claim 1, wherein the tissue factor apoprotein is a mutant of tissue factor apoprotein.

## Revendications

1. Procédé de réactivation de facteur tissulaire-apoprotéine, **caractérisé en ce que** le facteur tissulaire-apoprotéine est congelé en présence d'un lipide et d'un détergent, que le matériau congelé est décongelé, et que le mélange réactionnel, sans élimination ultérieure du détergent, est dilué avec un tampon, et est mis en oeuvre, additionné le cas échéant d'additifs, pour d'autres utilisations, comme agent thérapeutique ou comme agent diagnostique.

2. Procédé selon la revendication 1, **caractérisé en ce qu'**un lipide pur, un mélange de lipides purs ou un mélange naturel de lipides est utilisé.

3. Procédé selon la revendication 1, **caractérisé en ce que** le détergent est utilisé en une concentration de jusqu'à 50%.

4. Procédé selon la revendication 1, **caractérisé en ce que** l'on utilise un détergent non ionique.

5. Procédé selon la revendication 1, **caractérisé en ce que** l'on congèle à une température allant de 0°C à -200°C.

6. Procédé selon la revendication 1, **caractérisé en ce que** l'on utilise un facteur tissulaire-apoprotéine dissous.

7. Procédé selon la revendication 1, **caractérisé en ce que** la concentration du facteur tissulaire-apoprotéone s'élève à jusqu'à 50 mg/ml.

8. Procédé selon la revendication 1, **caractérisé en ce que** la concentration en lipide s'élève à jusqu'à 500 mg/ml.

9. Procédé selon la revendication 1, **caractérisé en ce que** le facteur tissulaire-apoprotéine est d'origine humaine, animale ou recombinée.

10. Procédé selon la revendication 1, **caractérisé en ce que** le facteur tissulaire-apoprotéine est un mutant du facteur tissulaire-apoprotéine.
